(19) Europäisches Patentamt
European Patent Office
Office européen des brevets

(11) Publication number: 0 165 044
A2

# (12) EUROPEAN PATENT APPLICATION

(21) Application number: 85304127.5

(22) Date of filing: 11.06.85

(51) Int. Cl.[4]: C 12 P 1/00
C 12 P 13/04, C 12 P 13/06
//C12M1/36

(30) Priority: 11.06.84 US 619116

(43) Date of publication of application:
18.12.85 Bulletin 85/51

(84) Designated Contracting States:
AT BE CH DE FR GB IT LI LU NL SE

(71) Applicant: GENEX CORPORATION
6110 Executive Boulevard
Rockville Maryland 20852(US)

(72) Inventor: Hsiao, Humg Yu
15937 Indian Hills Terrace Derwood
Maryland 20855(US)

(74) Representative: Hildyard, Edward Martin et al,
Frank B. Dehn & Co. European Patent Attorneys Imperial House 15-19 Kingsway
London WC2B 6UZ(GB)

## (54) Method of reacting an aldehyde- containing compound with a nucleophilic compound.

(57) The present invention relates to a method of reacting an aldehyde-containing compound with a nucleophilic compound in the presence of a catalyst, which comprises monitoring the pH of the reaction mixture and adjusting the rate of addition of the aldehyde-containing compound into the reaction mixture to prevent a substantial reduction in the pH of the reaction mixture below a determined level indicative of the accumulation of an undesirably high concentration of aldehyde containing compound, which results in the occurrence of unwanted side reactions.

EP 0 165 044 A2

0165044

# METHOD OF REACTING AN ALDEHYDE-CONTAINING COMPOUND WITH A NUCLEOPHILIC COMPOUND

## BACKGROUND OF THE INVENTION

The present invention relates to a method of controlling the rate of addition of an aldehyde-containing compound to a reaction mixture including a nucleophilic compound.

## DESCRIPTION OF THE BACKGROUND ART

A number of commercially important compounds can be synthesized by reacting aldehyde-containing compounds and nucleophilic compounds in the presence of an enzymatic catalyst. As examples, the enzyme serine hydroxymethyltransferase (SHMT) can catalyze the reaction of glycine with formaldehyde to form serine, with acetaldehyde to form allo-threonine and threonine, and with benzaldehyde to form β-phenyl-serine. Since the aldehyde-containing compounds can interact with and inactivate the catalyst, it is advantageous to keep the concentration of aldehyde-containing compound in the reaction mixture relatively low. This can be achieved by the gradual addition of the aldehyde-containing compound to the reaction mixture. On the other hand, the concentration of aldehyde-containing compound in the reaction mixture should be kept high enough to give a reasonable reaction rate. A pseudo-steady state should be achieved in which the rate of addition of aldehyde-containing compound and its rate of conversion are equal in order to maintain a constant level of aldehyde-containing compound in the reaction mixture. As the reaction proceeds to completion, the conversion rate generally slows and the feed rate must be slowed

accordingly, otherwise, the accumulation of the aldehyde-containing compound in the reaction mixture due to the imbalance between feed rate and conversion rate will result in inactivation of the biocatalyst. Therefore, a good monitoring system which can detect changes in the concentration of the aldehyde-containing compound in the reaction mixture is needed to control the rate of addition of the aldehyde-containing compound.

Heretofore, there has not been available a simple, continuous and sensitive monitoring system to control the rate of addition of aldehyde-containing compounds to reaction mixtures containing a nucleophilic compound, resulting in, for example, inefficient synthesis reactions and/or inactivation of catalysts.

There thus remains a need for a simple, continuous and sensitive method to monitor the level of aldehyde-containing compound and to control the rate of addition of aldehyde.

According to one aspect of the present invention, there is provided a method of reacting an aldehyde-containing compound with a nucleophilic compound in the presence of a catalyst, e.g. an enzyme, which comprises monitoring the pH of the reaction mixture and adjusting the rate of addition of the aldehyde-containing compound into the reaction mixture to prevent a substantial reduction in the pH of the reaction mixture below a determined level indicative of the accumulation of an undesirably high concentration of aldehyde-containing compound, which results in the occurrence of unwanted side reactions. The pH of the reaction mixture is preferably monitored continuously.

BRIEF DESCRIPTION OF THE DRAWINGS

FIG. 1 is a schematic diagram of a bioreactor and pH monitored feedback control system suitable for use with the method of the present invention.

FIG. 2 is a graphic depiction of L-serine production from glycine with pH controlled formaldehyde addition over a 23-hour period in accordance with the method of the present invention.

DETAILED DESCRIPTION OF THE PREFERRED EMBODIMENT

The method of the present invention can be employed in the synthesis of a desired compound from an aldehyde-containing compound and a nucleophile, wherein the desired reaction does not produce a net change in pH. Such reactions generally require catalysts to provide a favorable reaction rate.

Preferred aldehyde-containing compounds for use with the present invention include compounds of the formula R-CHO wherein R is alkyl, aryl, substituted aryl, alkenyl, alkynyl, and the like. Formaldehyde, acetaldehyde and benzaldehyde are particularly preferred aldehyde-containing compounds, with formaldehyde being most preferred for use with this invention.

Preferred nucleophilic compounds are primary amines, particularly amino acids. Glycine is a particularly preferred nucleophile.

In the synthesis of a desired compound from an aldehyde-containing compound and a nucleophile, certain undesirable side reactions may occur in the reaction mixture. For example, where the desired reaction is represented by the general reaction scheme

$$(1)\quad \text{R-CHO} + \text{compound A} \underset{}{\overset{\text{catalyst}}{\rightleftarrows}} \text{compound B},$$

wherein R-CHO is as defined above, compound A is the nucleophile (e.g., glycine), and compound B is the desired compound (e.g., serine), the following non-enzymatic reactions may occur in the reaction mixture

(2) compound Ap $\rightleftharpoons$ compound Aup + $H^+$ $\overset{R-CHO}{\rightleftharpoons}$ compound C

(3) compound Bp $\rightleftharpoons$ compound Bup + $H^+$ $\overset{R-CHO}{\rightleftharpoons}$ compound D

wherein compounds Ap and Aup represent the protonated and unprotonated forms of compound A respectively, compounds Bp and Bup represent the protonated and unprotonated forms of compound B respectively, compound C represents an Schiff base form of compound A (e.g., hydroxylmethyl glycine), and compound D represents an Schiff base form of compound B (e.g., hydroxylmethyl serine), R being as defined above.

Reaction (1) is the desired reaction and is thermodynamically favored when reactions (1), (2) and (3) coexist. Consequently, reactions (2) and (3) occur in significant amounts only in the presence of excess aldehyde. There is no acid or base formation in reaction (1), i.e., the desired reaction.

Reaction (2) is Schiff base formation. Compound A has a nucleophilic group, and this nucleophilic group has a protonated and an unprotonated form under the reaction conditions (pH and temperature) necessary for reaction 1. The method of this invention takes advantage of the fact that the undesired side reactions, indicative of inappropriately high aldehyde concentrations, can be detected by a drop in pH of the reaction mixture.

As an example, during the catalytic synthesis of serine from formaldehyde and glycine (reaction 1), the following undesirable side reactions may occur in the reaction mixture:

(2) protonated glycine $\rightleftharpoons$ unprotonated glycine + $H^+$ $\overset{HCHO}{\rightleftharpoons}$ hydroxymethyl glycine

(3) protonated serine $\rightleftharpoons$ unprotonated serine + $H^+$ $\overset{HCHO}{\rightleftharpoons}$ hydroxymethyl serine.

A drop in pH indicates an increase in the undesired side reactions (2) and (3) caused by excessive formaldehyde in the reaction mixture.

In order to increase reaction efficiency and/or prevent inactivation of catalyst by the aldehyde-containing compound, it is desirable to provide for the gradual addition of aldehyde-containing compound to the reaction mixture. Furthermore, it is desirable to maintain the concentration of aldehyde-containing compound at or below a certain level for the same reasons. In order to protect against deactivation of the catalyst by the aldehyde-containing compound, the concentration of aldehyde-containing compound can be maintained at a level below the level at which its concentration is highly toxic to the catalyst.

The pH of the reaction mixture is selected and maintained at a level which favors reaction (1). Since the feed solution of aldehyde-containing compound is generally slightly more acidic than is desired for the reaction mixture, its addition during the course of the reaction may interfere with the monitoring of pH changes caused by the undesired side reactions. Accordingly, it is desirable either to adjust the pH of the aldehyde-containing solution to that of the reaction mixture prior

to feeding or to offset its acidity by periodic additions of a base such as 10% KOH. Offsetting the acidity of the reaction mixture brought about by the presence of aldehyde-containing compound allows for the monitoring of pH drop due to undesirable side reactions (2) and (3).

Controlling the rate of addition of aldehyde-containing compound to the reaction mixture according to the present invention involves monitoring, generally continuously, the pH of the reaction mixture and selectively introducing aldehyde-containing compound into the reaction mixture to prevent a drop in reaction mixture pH indicating the presence of undesirable side reactions (2) and (3), which result from excess aldehyde concentration in the reaction mixture.

In utilizing the method of the present invention, the concentration of nucleophile can be higher than its saturation level. The pH of the reaction mixture is preferably from about 4 to about 11, since it is difficult to ascertain slight changes in pH outside that range and the catalyst might be inactivated. A pH within the range of from about 6 to about 8.5 is particularly preferred.

The invention will be described in greater detail in connection with the synthesis of L-serine from glycine and formaldehyde, but it is to be understood that the invention applies equally to other reactions involving aldehyde-containing compounds and nucleophiles, provided that the desired reaction is one which does not produce a net change in the pH of the reaction mixture.

Enzymatic synthesis of L-serine from glycine and formaldehyde generally requires the presence of the enzyme serine hydroxymethyltransferase (sometimes referred to as SHMT) and the cofactor tetrahydrofolate (sometimes referred to as THF or tetrahydrofolic acid). Formaldehyde (HCHO) reacts with THF to form methylene-THF. Glycine then reacts with methylene-THF in the presence of SHMT to form L-serine and regenerate THF. The synthesis advantageously takes place in a reaction vessel, for example, a stirred tank reactor, with the reaction mixture at a temperature of from about 4°C to about 60°C and at a pH of from about 4 to about 11. The preferred reaction conditions include carrying out the reaction at a temperature of from about 20 to about 45°C and a pH of about 6 to 8.5. If the temperature is below about 4°C, the reaction time is slowed considerably, and if the temperature rises above about 60°C the enzyme can be denatured. Similarly, at a pH below about 4 or higher than about 11, the enzyme can be inactivated. High speed of agitation of the mixture facilitates the dispersion of HCHO.

Serine hydroxymethyltransferase is very sensitive to formaldehyde. Formaldehyde at low concentration can quickly inactivate SHMT enzyme. Since formaldehyde is one of reactants necessary to make serine from glycine, the prevention of SHMT inactivation by formaldehyde is needed to have a successful process. THF is a cofactor for SHMT in the reaction; therefore, a catalytic amount of THF would normally be enough to carry reaction. THF is recycled in the reaction, shown in the following scheme.

$$\text{HCHO} + \text{THF} \longleftrightarrow \text{methylene-THF} + H_2O$$

$$H_2O + \text{methylene-THF} + \text{glycine} \overset{\text{SHMT}}{\longleftrightarrow} \text{serine} + \text{THF}$$

Net reaction

$$\text{HCHO} + \text{glycine} \longleftrightarrow \text{serine}$$

Since THF has a high affinity for HCHO (Keq = 3 x $10^4$), THF is used to consume HCHO in order to prevent HCHO from inactivating the SHMT. Therefore, a THF concentration higher than catalytic amount is employed to prevent enzyme inactivation by HCHO.

If desired, the concentration of THF may be brought to saturation levels, which are dependent upon pH and temperature. For example, at a pH of about 7.5 and a reaction temperature of about 37°C in an aqueous solution THF concentrations the excess of 50 mM may be achieved. Preferred THF concentrations range between 0.01 mM to 50 mM with a particularly preferred range between 0.1 mM and 5 mM. An excess presence of THF coupled with a gradual formaldehyde addition rate prevents formaldehyde accumulation and thus SHMT inactivation.

Tetrahydrofolate is very unstable in the presence of oxygen. A preferred method of preventing oxygen contact with the reaction mixture is by the induced presence of a non-reacting gas in the reaction vessel. Particularly preferred non-reacting gases are nitrogen and argon. Since a high rate of agitation is required for the reaction, it is extremely difficult to maintain an $O_2$-free environment. If THF is oxidized by air, the protection of SHMT from HCHO by reaction with THF no longer exists.

The SHMT is generally in the reaction mixture in the form of purified enzyme, crude extract or contained in whole cells of microorganisms which have been transformed with expression vectors containing DNA sequences encoding the amino acid sequence of SHMT, under the control of regulatory sequences which direct the expression of SHMT. The SHMT may be immobilized in the reactor as is well known in the art.

The instability of THF and thus SHMT under reaction conditions limits the efficiency of the enzymatic process for L-serine synthesis. The presence in the reaction mixture of a stabilizing amount of a reducing agent or a mixture of reducing agents stabilizes tetrahydrofolic acid and thus aids in the stabilization of serine

hydroxymethyl-transferase under reaction conditions, without inhibiting the enzymatic process. This stabilizing effect on THF is enhanced by prevention of air contact with the reaction mixture. The reducing agent is added until the redox potential of the reaction mixture is about -300mV or less, and added during the reaction to maintain the desired redox potential.

The formaldehyde concentration in the reaction mixture is preferably maintained at a level which is not significantly toxic to enzyme concentration. As the glycine is converted into L-serine, the formaldehyde is consumed and THF is regenerated. Formaldehyde is preferably added to the reaction mixture at a rate less than or equal to the rate at which it is consumed in the reaction. The precise rate of addition will vary with the reaction conditions (pH, temperature and enzyme concentration, etc.).

As the reaction continues and the concentration of L-serine increases in the reaction mixture, the reaction rate slows. If the rate of addition of the formaldehyde is not concomitantly reduced, then formaldehyde concentration in the reaction mixture increases. As formaldehyde concentration increases, the pH of the reaction mixture drops, since the free formaldehyde reacts with the amino group of glycine and/or serine as shown in the following reversible reaction scheme.

$$NH_3^{+}-R \rightleftharpoons H_2N-R + H^{+}$$

$$HCHO + H_2N-R \rightleftharpoons R-\overset{H}{N}-\underset{H}{\overset{H}{C}}-OH$$

If no further formaldehyde is introduced to the reaction mixture, or the rate of introduction is sufficiently slowed, the above reaction will reverse, and pH will go up. The rate of this reversed reaction (and the rate of pH increase) is dependent upon the rate of conversion of glycine to serine.

In accordance with the present invention, an increase in formaldehyde concentration in the reaction mixture is monitored and controlled by continuously measuring the pH and selectively adjusting the rate of introduction of formaldehyde into the reaction mixture to prevent the pH level from dropping below the reaction pH, thereby indicating an increase in formaldehyde concentration. At a typical reaction pH of 6.5-7.0, the maximum tolerated drop in pH is about 0.2. It is preferable to regulate introduction of formaldehyde based on a pH drop within the range of from about 0.05 to about 0.1, depending on the sensitivity of pH controller.

The pH is monitored using conventional means (e.g., pH meter) which are preferably connected to a conventional pH controller. HCHO feeding pump is connected to the "acid" position of this controller. A drop in pH to the preset lower limit of the reaction pH indicates an increase in formaldehyde concentration, and the controller will automatically turn the HCHO feeding pump off. Therefore, it ensures that there is no more HCHO being fed into the system. At this point, HCHO feeding rate can be manually or automatically reduced (the extent of reduction can go from 5%-95% of its original value, preferably 10% - 40%). Once no more HCHO was fed into the reaction mixture, pH will go up again. When it goes higher than its preset value, controller senses this and turns the HCHO feeding pump on.

Since the pH of a 37% formaldehyde concentration is about 4.5, it is preferred that the pH of this HCHO

solution be adjusted to a level near a neutral pH (from about 4.5 to about 6-7.0) by addition of a catalytically acceptable base prior to the initial introduction of formaldehyde to the mixture. A pH drop due to undesirable side reactions may thereby more easily be monitored.

The invention is further illustrated by the following examples, which are not intended to be limiting.

## EXAMPLE I

Glycine (2M, final concentration) was dissolved in water. The pH of the reaction mixture was about 5.84-5.88. Twenty cc of this glycine solution was placed in a beaker with stirring. The pH was monitored by a pH meter Chemcadet pH meter/controller, Cole-Parmer. NaOH (10%) was used to raise the pH of the glycine solution to its initial value as indicated below. HCHO (1N) was added and decrease in pH was recorded.

Results are shown in Table 2:

Table 2

| pH (initial) | HCHO | pH (final) | Δ pH | ΔpH/mM HCHO |
|---|---|---|---|---|
| 6.54 | 5mM (1st addition) | 6.24 | 0.3 | 0.06 |
| | 5mM (2nd addition) | 5.96 | 0.58 | 0.058 |
| 7.03 | 5mM | 6.8 | 0.23 | 0.046 |
| | 5mM | 6.56 | 0.47 | 0.047 |
| 7.5 | 5mM | 7.38 | 0.12 | 0.024 |
| | 5mM | 7.24 | 0.26 | 0.026 |

The results demonstrate that formaldehyde addition to a glycine solution causes a drop in pH and indicate the sensitivity of the monitoring system.

EXAMPLE II

Conditions used were identical to ones in Example I except that the glycine concentration was 1M. Results are shown in Table 3.

Table 3

| pH (initial) | HCHO | pH (final) | Δ pH | Δ pH/mM HCHO |
|---|---|---|---|---|
| 6.56 | 5mM | 6.26 | 0.3 | 0.060 |
| | 5mM | 6.00 | 0.56 | 0.056 |
| 7.09 | 5mM | 6.89 | 0.2 | 0.04 |
| | 5mM | 6.66 | 0.43 | 0.043 |
| 7.5 | 5mM | 7.38 | 0.12 | 0.024 |
| | 5mM | 7.23 | 0.27 | 0.027 |

The results demonstrate that formaldehyde addition to a glycine solution lowers pH. The sensitivity of the monitoring system was similar to that of Example I, in which the glycine concentration was higher.

EXAMPLE III

Conditions used were identical to the ones in Example I except that 30ml of a mixture of 2M serine and 0.8M glycine was used in order to approximate conditions occurring near the end of the reaction of formaldehyde and glycine to produce serine. Results are shown in Table 4.

Table 4

| pH (initial) | HCHO | pH (final) | Δ pH | Δ pH/mM HCHO |
|---|---|---|---|---|
| 6.50 | 5mM | 6.31 | 0.19 | 0.038 |
| | 5mM | 6.14 | 0.36 | 0.036 |
| 7.03 | 5mM | 6.89 | 0.14 | 0.028 |
| | 5mM | 6.78 | 0.25 | 0.025 |
| 7.5 | 10mM | 7.36 | 0.14 | 0.014 |
| | 10mM | 7.20 | 0.3 | 0.015 |

The results demonstrate that formaldehyde addition to a mixture of glycine and serine in solution causes a drop in pH.

EXAMPLE IV

Conditions used were identical to the ones in Example I except that acetaldehyde was used. Results were shown in Table 5:

Table 5

| pH (initial) | Acetaldehyde | pH (final) | Δ pH | Δ pH/mM Acetaldehyde |
|---|---|---|---|---|
| 7.00 | 8.9mM | 6.85 | 0.15 | 0.017 |
| | 8.9mM | 6.72 | 0.28 | 0.016 |
| 7.59 | 8.9mM | 7.47 | 0.12 | 0.013 |
| | 8.9mM | 7.38 | 0.21 | 0.012 |
| 8.02 | 8.9mM | 7.94 | 0.08 | 0.009 |
| | 8.9mM | 7.86 | 0.16 | 0.009 |

EXAMPLE V

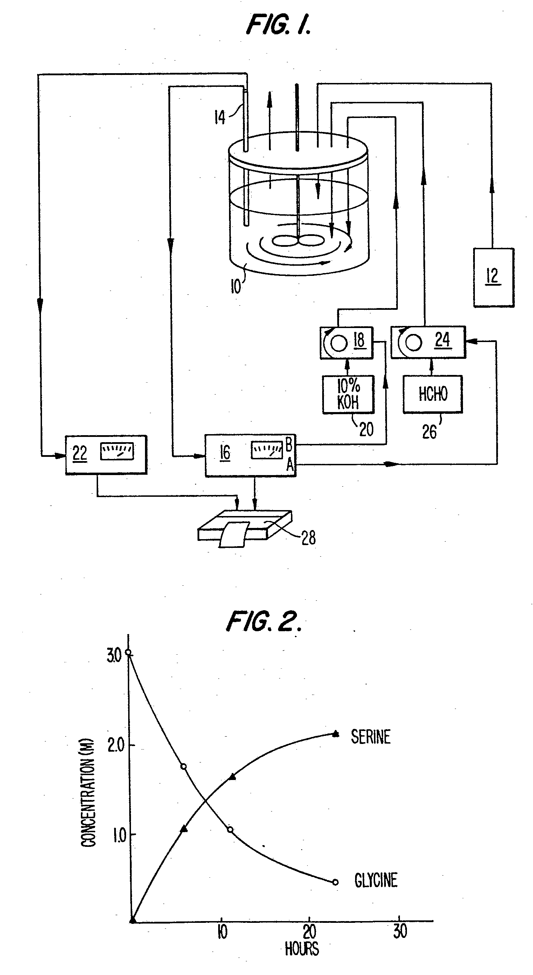

A 150ml bioreactor 10 (see Fig. 1) with 100ml working volume, containing 22.5 g glycine (3M) 0.5mM pyridoxal-phosphate and enzyme serine hydroxymethyltransferase (450 units) was sealed and blanketed with $N_2$ from $N_2$ tank 12. The temperature was maintained at 37°C. The bioreactor was equipped with a conventional combined pH and redox electrode 14. The pH probe was connected to a conventional pH controller 16. A base pump 18 was connected to a "base" position of controller. The set point at base position of controller was pH 6.5. The pH of the reaction mixture was adjusted and maintained at 6.5 by introduction of 10% KOH from KOH tank 20. The redox potential was maintained using a conventional redox controller 22, and β-Mercaptoethanol (0.2ml) was introduced to lower the redox potential of reaction mixture below -300mV. Tetrahydrofolate (1mM, final concentration) was added after the redox potential and pH were reached and stabilized at the desired value. Formaldehyde pump 24 was connected to the acid position of pH controller. The set point at acid position of the controller was pH 6.5. Reaction started by pumping formaldehyde from formaldehyde tank 26 with an initial formaldehyde addition rate of 1.7 ml/hr. In the first 4 to 5 hours of reaction, there was little change of pH. When formaldehyde conversion rate became slower than formaldehyde addition rate due to the accumulation of serine, a slight build up of formaldehyde reacted with amino group of serine and/or glycine to cause pH drop (see Example I-III). When pH dropped to 6.44, the controller automatically turned the formaldehyde pump off. Once no further formaldehyde was introduced, pH went up. When it reached 6.48, controller automatically turned the formaldehyde pump on. At this time (or right after pH dropped to 6.44), formaldehyde feeding rate was

decreased to 1.2 ml/hr. After the pH remained stable for a few hours, the pH dropped again and formaldehyde pump stopped. The whole process was repeated until a desired level of conversion was reached. Data was recorded from the pH controller 16 and redox potential controller 22 by a conventional two channel recorder 28. During the course of this reaction, 0.08 ml of 10% KOH was added every hour to offset the acidity of added formaldehyde. Result of the reaction with pH controlled formaldehyde addition is shown in Figure 2. After correcting the dilution factor (1.13), an 82.5% (mole/mole) conversion was obtained in 23 hours with an average productivity of 10.9 gram serine formation per hour per liter. Enzyme activity remained at 90% at the end of the reaction.

EXAMPLE VI

Conditions used to make serine from glycine and formaldehyde by enzyme (serine hydroxymethyltransferase) were identical to conditions in Example IV except that the set point was pH 7.0 (instead of 6.5). After 24 hours of reaction, an 80% conversion (mole/mole) was obtained with an average productivity of 10.5 gram serine formation per hour per liter. Enzyme activity remained at 92% at the end of the reaction.

CLAIMS:

1. A method of reacting an aldehyde-containing compound with a nucleophilic compound in the presence of a catalyst, characterised in

that the pH of the reaction mixture is monitored and the rate of addition of the aldehyde-containing compound into the reaction mixture is adjusted to prevent a substantial reduction in the pH of the reaction mixture below a determined level indicative of the accumulation of an undesirably high concentration of aldehyde containing compound.

2. A method of reacting an aldehyde-containing compound with a nucleophilic compound in the presence of a catalyst which comprises introducing an aldehyde-containing compound into a reaction mixture containing a nucleophilic compound and a catalyst, terminating introduction of the aldehyde-containing compound when a drop in the pH level of the reaction mixture occurs which indicates an excessive concentration of aldehyde-containing compound, and reintroducing aldehyde-containing compound to the reaction mixture when the pH level of the reaction mixture returns to the desired reaction pH.

3. A method as claimed in claim 1 or claim 2 wherein the catalyst is an enzyme.

4. A method as claimed in any of claims 1 to 3 wherein the aldehyde-containing compound is selectively introduced to the reaction mixture to prevent a drop in reaction pH of greater than about 0.2.

5. A method as claimed in claim 4 wherein the aldehyde-containing compound is selectively introduced to the reaction mixture to prevent a drop in reaction pH of greater than about 0.05 to about 0.1.

6. A method as claimed in any one of the preceding claims wherein the aldehyde-containing compound is a compound of formula R-CHO wherein R is alkyl, aryl, substituted aryl, alkenyl or alkynyl.

7. A method as claimed in claim 6 wherein the aldehyde-containing compound is formaldehyde, acetaldehyde or benzaldehyde.

8. A method as claimed in any one of the preceding claims wherein the nucleophilic compound is an amine-containing compound.

9. A method as claimed in claim 8 wherein the nucleophilic compound is an amino acid.

10. A method as claimed in claim 9 wherein the nucleophilic compound is glycine.

11. A method as claimed in claim 10 wherein the reaction mixture futher includes tetrahydrofolic acid and the catalyst is serine hydroxymethyltransferase.

12. The method of claim 10 wherein the reaction mixture includes glycine, formaldehyde and tetrahydrofolic acid and the catalyst is serine hydroxymethyltransferase.

13. A method as claimed in any one of the preceding claims wherein the pH of the aldehyde-containing solution is adjusted substantially to the pH of the reaction mixture prior to addition, or, to neutralise the acidity of the added aldehyde compound, a base is periodically added to the reaction mixture.

14. A method as claimed in claim 13 wherein potassium hydroxide solution is periodically added to the reaction mixture.

15. Apparatus for performing the method of claim 1 comprising a reactor vessel (10), means (26) for supplying an aldehyde-containing compound to said reactor vessel, means (14) for monitoring the pH of the reaction mixture in said vessel, means (16) for setting a determined pH, means (16) for comparing said monitored pH with said determined pH, and means (24) for interrupting or reducing supply of said aldehyde-containing compound to said reactor vessel when said monitored pH falls below said determined pH.

0165044

1/1

FIG. 1.

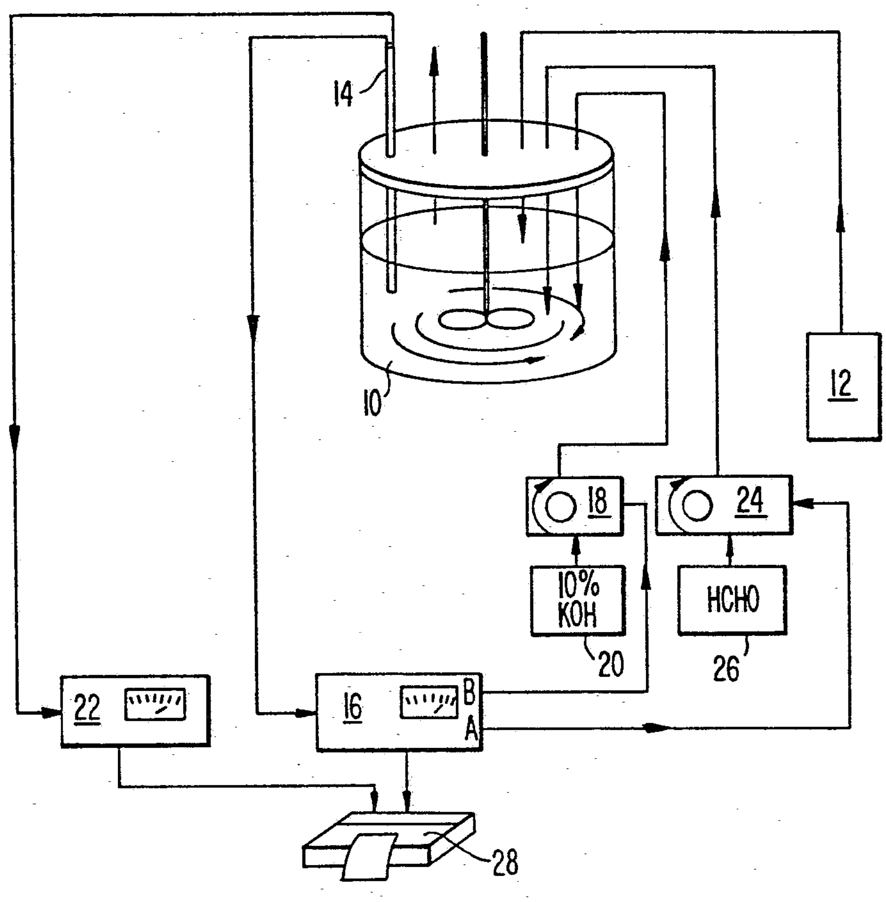


FIG. 2.